Europäisches Patentamt

(19) European Patent Office

Office européen des brevets

(11) Publication number: **0 057 451**
**B1**

(12) **EUROPEAN PATENT SPECIFICATION**

(45) Date of publication of patent specification: **27.12.84**

(51) Int. Cl.³: **A 61 N 1/04**

(21) Application number: **82100644.2**

(22) Date of filing: **01.02.82**

(54) **Body implantable cushioned lead.**

(30) Priority: **02.02.81 US 230729**

(43) Date of publication of application:
**11.08.82 Bulletin 82/32**

(45) Publication of the grant of the patent:
**27.12.84 Bulletin 84/52**

(84) Designated Contracting States:
**DE FR GB NL**

(56) References cited:
**DE-A-3 006 472**
**US-A-3 476 116**
**US-A-4 011 861**
**US-A-4 156 429**
**US-A-4 230 604**

(73) Proprietor: **MEDTRONIC, INC.**
**3055 Old Highway Eight**
**Minneapolis Minnesota 55440 (US)**

(72) Inventor: **Juncker, David F.**
**16 E.Minnehaha Parkway**
**Minneapolis, Minn.55427 (US)**
Inventor: **Cahalan, Patrick T.**
**11833 Jersey**
**Champlin, Minn.55316 (US)**
Inventor: **Coury, Arthur J.**
**2225 Hillside Avenue**
**St. Paul, Minn.55108 (US)**

(74) Representative: **Schwan, Gerhard, Dipl.-Ing.**
**Elfenstrasse 32**
**D-8000 München 83 (DE)**

Courier Press, Leamington Spa, England.

## Description

Background of Prior Art

Electrical stimulation of the body for medical purposes is well known in the prior art. An example of a device for this purpose is the well-known cardiac pacemaker. In the pacemaker context, as well as other body stimulation contexts, the stimulation is delivered to the desired body site by an electrode component of a lead.

A variety of body implantable electrical leads for the delivery of electrical stimulation energy to a desired body site are known in the prior art. Recent years have seen increased usage of transvenous lead which can deliver the stimulation energy to the desired body site while significantly lowering patient risk and morbidity. For example, the use of a transvenous/endocardial lead eliminates the necessity of a thoracotomy while delivering stimulation energy to the heart to provide a pacemaking function. Such endocardial leads may be used for sensing as well as pacing.

In many stimulation and sensing contexts, maintenance of a reliable electrical contact at the desired electrode site has proven difficult. Interactions between the lead and the body at the contact site can vitiate the desired effects of the stimulation. For example, material reactions may encourage fibrosis. In the pacemaking context, fibrosis is believed to be a major factor in the increase in chronic threshold that is usually experienced. Also, mechanical trauma may result in inflammation of the tissue to be stimulated. Such inflammation may alter the response of the tissue to the stimulation energy, both acutely and chronically.

For example, in chronic use of such leads, it has been observed that the contractions of the heart against the distal end of the lead and the axial mechanical pressure applied thereto may traumatize the tissue in contact with the distal end. The traumatized tissue may form scar tissue around the electrode(s) to reduce the effect of stress placed upon the endocardium below the tissue damage limit. In chronic experience, it is observed that the threshold currents sufficient to stimulate the heart increase as the scar tissue is formed until the thickness of the scar tissue stabilizes. The required energy to stimulate the heart is thereby increased because of the additional potential drop across the nonresponsive cardiac cells. A discussion of this mechanism in conjunction with the realization of optimum electrode surface area appears in the paper entitled "Comparison of Power Sources for Advanced Pacemaker Applications" by Rasor, Spickler and Clabaugh, *Proceedings of the Seventh Intersociety Energy Conversion Engineering Conference*, January 1972, pages 752—760.

Other interactions between the lead and body at the contact site, while not directly affecting the response of the tissue to the stimulation energy, can result in the ocurrence of undesirable events. In some circumstances where electrical stimulation of body tissue is indicated, the tissue to be stimulated is irritable. The placement of a pacemaking lead may induce a cardiac arrhythmia. The presence of the lead may also promote thrombus formation.

Pressure induced trauma are known to be the leading cause of enlarged fibrotic encapsulation of the electrode tip.

Cardiac tissue is occluded under the tip affecting a diffusion barrier to oxygen and other nutrients carried by the blood while also being deprived of the ability to transfer waste products from the tissue into the blood. A large number of tissue cells are so occluded by existing endocardial electrode tips. Cells of heart tissue must communicate with the blood in the ventricles of the heart by way of diffusion due to the lack of capillaries near the endocardial surface.

The interactions noted above have long been recognised and efforts have been made to ameliorate their consequences. For example, leads have been configured to reduce mechanical trauma and the response of irritable tissue during lead placement. Materials have been selected for the lead body and electrodes to minimize fibrosis. Thrombus formation may also be countered by the administration of suitable drugs.

The administration of drugs to counter the undesirable interactions between the lead and the body noted above has not gained widespread acceptance in that it has heretofore required a systemic treatment to counter a localized interaction. Also, lead configuration must take into account other factors such as the efficiency of the delivery of the stimulation energy, the ease of lead placement, maintenance of the desired electrode position and reliability of the lead over extended periods of time. An accommodation of these interests has resulted in leads whose configuration necessarily results in undesirable interactions between the lead and body.

Brief Summary of the Invention

The present invention provides a means for adapting presently available body implantable leads so as to ameliorate the effects of undesirable interactions between the lead and the contacted tissue. This is accomplished by providing the electrode contact surface with a cushioning body of a soft, compliant, water swellable, polymeric, water insoluble material inert to body tissues and fluids. The material consists essentially of a hydrogel which is ionically conductive. The hydrogel is permeable to water, oxygen, $CO_2$ and the like so as to allow their diffusion through the cushioning body both to and away from the occluded tissue cells at the contact site. The water-permeable hydrogel body is ionically conductive, thereby providing electrical communication between the metal electrode and the tissue. Lastly, due to the cushioning effect provided by the hydrogel body, a barrier is provided between the metal electrode and the tissue which is capable of reducing mechanical stresses applied to the heart tissue thereby minimizing trauma effects at the contact site. DE—A—3 006 472 discloses an electrode covered with ion-conducting membranes which prevent changes in salt concentration in body tissue rather than provide a cushioning effect.

Brief Description of the Drawings

Figure 1 illustrates a portion of a body implantable lead constructed in accordance with the preferred embodiment of the present invention.

Figure 2 is an end view of the body implantable lead shown in Figure 1.

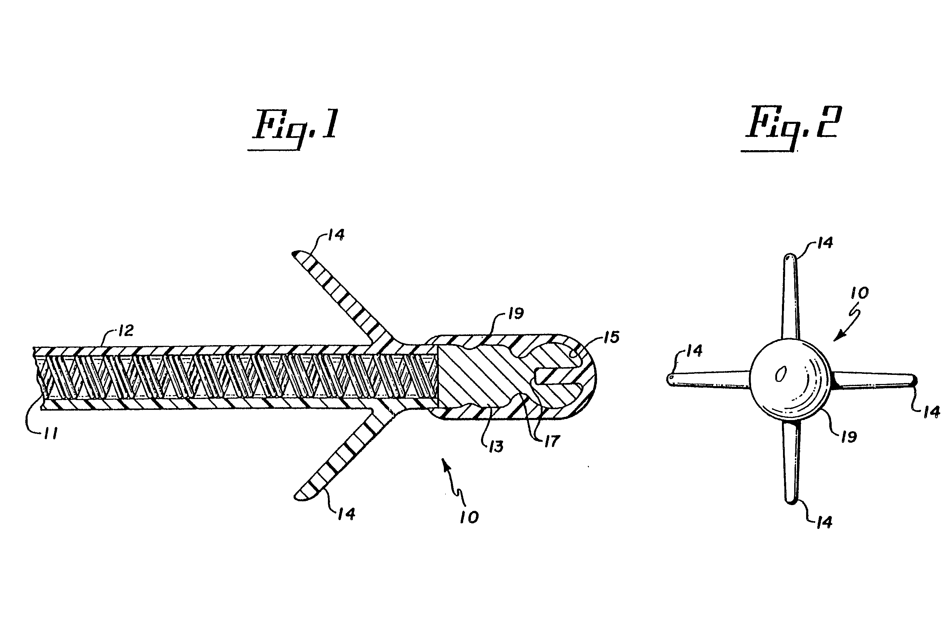

Detailed Description of the Invention

Referring to the Figures, there is illustrated a body implantable lead of the transvenous type, constructed in accordance with the invention. The lead body, generally designated 10, is comprised of a coiled, flexible conductor 11 and an overlying sheath 12. Coiled, flexible conductor 11 may be of any known design and preferably has a central aperture which will acept a stylet (not shown) to provide stiffness during lead placement, in known manner. The lead body may also be non-linear such that stylet insertion will straighten the lead body to facilitate transvenous placement, also in known manner. To facilitate the flexibility requirements of the present invention as well as for reliability considerations, flexible conductor 11 may be a multi-filar member, three filars being illustrated in the drawing. Conductor 11 is in electrical communication with a tip electrode 13 and a source of stimulation energy (not shown) for the delivery of stimulation energy to the desired body site. Sheath 12 may be a preformed tubular member, preferably of a body compatible polyurethane, to overlie conductor 11 and provide electrical insulation therefor, in known manner. Also, as known in the art, lead body 10 may be tined as indicated at 14 for purposes of fixation upon implantation.

In the prior art, many body stimulation leads are formed of a molded silicone. A preferable material for sheath 12 and lead body 10 is a body-compatible polyurethane. Such polyurethanes meet the flexibility requirements of transvenous leads and are typically less thrombogenic than silicone and more resistant to tearing and cutting. In general, the physical characteristics of polyurethane are more suited to transvenous leads than those of silicone, although silicone or any other body compatible material may be employed in practicing the present invention.

Also, as is known in the art, metal electrode 13 may be provided in various configurations. Additionally, various metals may be used, such as stainless steel and titanium. A preferred platinum or platinum alloy tip electrode 13 provides a contact surface 15 as shown in the Figures. Heretofore contact surface 15 has for the most part contacted the tissue at the desired body site directly. Cushioning body 19 is carried and positioned on contacting surface 15 of electrode tip 13 so as to interpose between electrode tip 13 and the body tissue to be contacted. Present commercially available platinum leads may be modified according to the invention by simply attaching a cushioning body to the electrode contact surface or surfaces. Attachment may be accomplished by adhesive bonding, for example as promoted by silane adhesion promoters. A mechanical interlock may also be used. In the drawing, tip 13 is modified by forming invaginations 17 therein to provide attachment by means of mechanical interlock.

Cushioning body 19 consists essentially of a hydrogel thick enough to allow for flexible contact between electrode tip 13 and the tissue contact site. Typically, thicknesses will range from about 1 micrometer to 1 millimeter. Such cushioning body has been found to provide low mechanical trauma and high sensing and stimulation effectiveness with endocardial tissue.

Cushioning body 19 acts to dampen lead body pressure and side-to-side motion between the electrode and the endocardial wall or other selected body contact site. The cushioning body thus enables a flexible attachment to develop between the electrode and the underlying tissue. This flexible attachment or link reduces mechanically induced trauma between tissue and the electrode metal tip which is believed to be a cause of increased thickness in the fibrotic encapsulation of electrodes.

Hydrogel polymers are ionically conductive, soft, compliant, water swellable and inert to body tissues and fluids. Cushioning body 19 is also ionically conductive and permeable thereby allowing electrical communication between electrode tip 13 and the contacted tissue as well as providing a diffusion path to and away from the occluded tissue site.

Generally speaking, a hydrogel having the properties above described will comprise a coherent, three-dimensional polymeric network capable of imbibing water without dissolving. usually, insolubility in water is provided by a crosslinked structure in the polymer. Hydrogels or water-containing gels may be comprised of water and various chemical substances including gelatin, polysaccharides, crosslinked acrylamide polymers, hydroxyethylmethacrylate polymers, crosslinked polyhydroxyethylacrylate, polymerized, crosslinked 2-acrylamido-2-methylpropane sulfonic acid or one of its salts such as the sodium or potassium type, crosslinked polyvinylpyrrolidone, polyacrylic acid, copolymers of the aforementioned monomers with each other and copolymers of the aforementioned monomers with other monomers such as styrene or other nonhydrogel type monomers. Hydrogels of low conductivity may be made conductive for purposes of this invention by incorporating salts such as sodium chloride or potassium chloride and like electrolytic salts in the hydrogel. This is most easily accomplished by dissolving a salt in a monomer-water-initiator solution and polymerizing. Other methods of preparation may also be used.

The preferred specific hydrogels are crosslinked polyacrylamide and crosslinked polymerized 2-acrylamido-2-methylpropane sulfonic acid or one of its salts. Volume 15, pages 273—291 of the *Encyclopedia of Polymer Science Technology* (1971), John Wiley Publishers, provides a section entitled HYDROGELS which describes the preparation of a variety of water imbibing polymers. The content thereof is incorporated herein by reference.

Example — Polyacrylamide Hydrogel The following ingredients were dissolved in 52.5 grams of deionized water: 40.0 grams acrylamide, 2.0 grams sodium chloride, 8.0 cc of 1% aqueous solution of methylene-bis-acrylamide. Nitrogen was vigorously bubbled through the resultant solution for 2.5 minutes. The following reagents were added to the solution simultaneously with stirring: 0.5 cc of a .38% solution of potassium bisulfite, 0.5 cc of a .38% solution of potassium persulphate, and 0.5 cc of a .24% solution of ferrous sulfate.

A portion of the resultant mixture may be withdrawn into syringe and ejected into the cavity formed by a mold surrounding electrode tip 13 under a nitrogen atmosphere. The mixture is then cured to form the hydrogel by allowing it to stand under the nitrogel atmosphere at room temperature until solidified. The mold is then removed.

A wide variety of lead configurations, including bipolar as well as unipolar, and cushioning body configurations consistent with the tissue contacting requirements of the lead and with the motion-absorbing requirements of the present invention may be utilized. It is, therefore, to be understood that, within the scope of the appended claims, the invention may be practiced otherwise than as specifically described.

Some of the subject-matter disclosed herein is claimed in our European patent application, no. 57 450.

**Claims**

1. A body-implantable lead (10) including a lead body having a distal end and at least one metal electrode (13) carried by the lead body at the distal end, the electrode including a contact surface (15) adapted to contact living tissue, wherein the lead further comprises a trauma-reducing, cushioning body (19) of a soft, compliant, water-swellable, polymeric, water-insoluble material inert to body tissues and fluids, the material consisting essentially of a hydrogel which is ionically conductive, the body being carried and positioned on the contact surface of the electrode so as to be interposed between it and the body tissue to be contacted whereby flexible connection between the electrode and the tissue is established.

2. The body-implantable lead of claim 1 wherein the electrode contact surface is at the tip of the lead and the hydrogel cushioning body is carried on the tip.

3. The body-implantable lead of claim 1 wherein the electrode metal is platinum or an alloy thereof.

4. The body-implantable lead of claim 1 wherein the hydrogel includes a crosslinked polymer.

5. The body-implantable lead of claim 1 wherein the hydrogel includes a copolymer.

6. The body-implantable lead of claim 1 wherein the hydrogel includes an acrylamide polymer.

7. The body-implantable lead of claim 6 wherein the acrylamide polymer is crosslinked.

8. The body-implantable lead of claim 1 wherein the hydrogel includes a methylacrylate polymer.

9. The body-implantable lead of claim 1 wherein the hydrogel includes a crosslinked ethylacrylate polymer.

10. The body-implantable lead of claim 1 wherein the hydrogel includes a crosslinked polymer of 2-acrylamido-2-methylpropane sulfonic acid or one of its salts.

11. The body-implantable lead of claim 10 wherein the polymer is a copolymer.

12. The body-implantable lead of claim 1 wherein the hydrogel includes a crosslinked vinylpyrrolidone polymer.

13. The body-implantable lead of claim 1 wherein the hydrogel includes a copolymer.

14. The body-implantable lead of claim 13 wherein the copolymer is comprised of at least two copolymerized hydrogel-forming monomers.

15. The body-implantable lead of claim 13 wherein the copolymer is comprised of at least one hydrogel-forming monomer and one non-hydrogel-forming monomer which are copolymerized.

16. The body-implantable lead of claim 1 wherein the lead is of the transvenous type.

17. The body-implantable lead of claim 1 wherein the lead is of the bipolar type.

**Revendications**

1. Conducteur (10) implantable dans le corps comprenant un corps de conducteur ayant une extrémité distale et au moins une électrode métallique (13) supportée par le corps de conducteur à l'extrémité distale, l'électrode comportant une surface de contact (15) destinée à être en contact avec des tissus vivants, dans lequel le conducteur comporte également une pièce de rembourrage (19) de réduction des traumatismes d'une matière souple, élastique, gonflable à l'eau, polymère, insoluble dans l'eau et inerte aux tissus et fluides du corps, la matière consistant essentiellement en un hydrogel qui est conducteur des ions, la pièce étant supportée et positionnée sur la surface de contact de l'électrode de manière à être intercalée entre elle et le tissu du corps qui doit être mis en contact, de manière à établir une connexion souple entre l'électrode et les tissus.

2. Conducteur implantable dans le corps selon la revendication 1, dans lequel la surface de contact d'électrode se trouve à l'extrémité du conducteur et la pièce de rembourrage en hydrogel est supportée sur l'extrémité.

3. Conducteur implantable dans le corps selon la revendication 1, dans lequel le métal de l'électrode est du platine ou un de ses alliages.

4. Conducteur implantable dans le corps selon la revendication 1, dans lequel l'hydrogel contient un polymère réticule.

5. Conducteur implantable dans le corps selon la revendication 1, dans lequel l'hydrogel contient un copolymère.

6. Conducteur implantable dans le corps selon la revendication 1, dans lequel l'hydrogel contient un polymère d'acrylamide.

7. Conducteur implantable dans le corps selon la revendication 6, dans lequel le polymère d'acrylamide est réticulé.

8. Conducteur implantable dans le corps selon la revendication 1, dans lequel l'hydrogel contient un polymère de méthylacrylate.

9. Conducteur implantable dans le corps selon la revendication 1, dans lequel l'hydrogel contient un polymère d'éthylacrylate réticulé.

10. Conducteur implantable dans le corps selon la revendication 1, dans lequel l'hydrogel contient un polymère réticulé d'acide sulfonique de 2-acrylamido-2-méthylpropane ou l'un de ses sels.

11. Conducteur implantable dans le corps selon la revendication 10, dans lequel le polymère est un copolymère.

12. Conducteur implantable dans le corps selon la revendication 1, dans lequel l'hydrogel contient un polymère de vinylpyrrolidone réticulé.

13. Conducteur implantable dans le corps selon la revendication 1, dans lequel l'hydrogel contient un copolymère.

14. Conducteur implantable dans le corps selon la revendication 13, dans lequel le copolymère est constitué par au moins deux monomères formant un hydrogel, copolymérisés.

15. Conducteur implantable dans le corps selon la revendication 13, dans lequel le copolymère est constitué par au moins un monomère formant un hydrogel et un monomère ne formant pas d'hydrogel, qui sont copolymérisés.

16. Conducteur implantable dans le corps selon la revendication 1, dans lequel le conducteur est du type transveineux.

17. Conducteur implantable dans le corps selon la revendication 1, dans lequel le conducteur est du type bipolaire.

**Patentansprüche**

1. Körperimplantierbare Leitung (10) mit einem ein distales Ende aufweisenden Leitungskörper und mindestens einer von dem Leitungskörpers an dem distalen Ende getragenen Metallelektrode (13), die eine mit lebendem Gewebe in Kontakt bringbare Kontaktfläche (15) hat, wobei die Leitung des weiteren mit einem traumareduzierenden Polsterkörper (19) aus einem gegenüber Körpergewebe und -medien inerten weichen, nachgiebigen, in Wasser aufquellbaren, polymeren, wasserunlöslichen Material versehen ist, das Material im wesentlichen aus einen Hydrogel besteht, das ionenleitend ist, und der Körper auf der Kontaktfläche der Elektrode derart getragen und positioniert ist, daß er zwischen die Kontaktfläche und das zu kontaktierende Körpergewebe zu liegen kommt, wodurch eine flexible Verbindung zwischen der Elektrode und dem Gewebe hergestellt wird.

2. Körperimplantierbare Leitung nach Anspruch 1, bei der die Elektrodenkontaktfläche an der Spitze der Leitung sitzt und der Hydrogel-Polsterkörper auf der Spitze abgestützt ist.

3. Körperimplantierbare Leitung nach Anspruch 1, bei der als Elektrodenmetall Platin oder eine Platinlegierung vorgesehen ist.

4. Körperimplantierbare Leitung nach Anspruch 1, bei der das Hydrogel ein vernetztes Polymer aufweist.

5. Körperimplantierbare Leitung nach Anspruch 1, bei der das Hydrogel ein Copolymer aufweist.

6. Körperimplantierbare Leitung nach Anspruch 1, bei der das Hydrogel ein Acrylamidpolymer aufweist.

7. Körperimplantierbare Leitung nach Anspruch 6, bei der das Acrylamidpolymer vernetzt ist.

8. Körperimplantierbare Leitung nach Anspruch 1, bei der das Hydrogel ein Methylacrylatpolymer aufweist.

9. Körperimplantierbare Leitung nach Anspruch 1, bei der das Hydrogel ein vernetztes Äthylacrylatpolymer aufweist.

10. Körperimplantierbare Leitung nach Anspruch 1, bei der das Hydrogel ein vernetztes Polymer aus 2-Acrylamid-2-methylpropansulfonsäure oder eines ihrer Salze aufweist.

11. Körperimplantierbare Leitung nach Anspruch 10, bei der das Polymer ein Copolymer ist.

12. Körperimplantierbare Leitung nach Anspruch 1, bei der das Hydrogel ein vernetztes Vinylpyrrolidonpolymer aufweist.

13. Körperimplantierbare Leitung nach Anspruch 1, bei der das Hydrogel ein Copolymer aufweist.

14. Körperimplantierbare Leitung nach Anspruch 13, bei der das Copolymer aus mindestens zwei copolymerisierten, Hydrogel bildenden Monomeren besteht.

15. Körperimplantierbare Leitung nach Anspruch 13, bei der das Copolymer aus mindestens einem Hydrogel bildenden Monomer und einem nicht Hydrogel bildenden Monomer besteht, die copolymerisiert sind.

16. Körperimplantierbare Leitung nach Anspruch 1, wobei die Leitung eine transvenöse Leitung ist.

17. Körperimplantierbare Leitung nach Anspruch 1, wobei die Leitung eine bipolare Leitung ist.

_Fig.1_

_Fig.2_

0057451